(19) Europäisches Patentamt
European Patent Office
Office européen des brevets

(11) Publication number: **0 192 068**
**B1**

(12) **EUROPEAN PATENT SPECIFICATION**

(45) Date of publication of patent specification: **23.05.90**

(51) Int. Cl.$^5$: **A 61 L 27/00,** C 08 G 69/10

(21) Application number: **86100784.7**

(22) Date of filing: **22.01.86**

(54) **Hard tissue prosthetics and process for the preparation thereof.**

(30) Priority: **19.02.85 US 702998**
**19.02.85 US 702999**

(43) Date of publication of application:
**27.08.86 Bulletin 86/35**

(45) Publication of the grant of the patent:
**23.05.90 Bulletin 90/21**

(84) Designated Contracting States:
**DE FR GB IT NL SE**

(56) References cited:
**EP-A-0 011 528**
**CH-A- 643 732**
**FR-A-2 287 242**
**FR-A-2 350 826**
**FR-A-2 364 644**

(73) Proprietor: **THE DOW CHEMICAL COMPANY**
**2030 Dow Center Abbott Road P.O. Box 1967**
**Midland Michigan 48640-1967 (US)**

(72) Inventor: **Dorman, Linneaus C.**
**2506 Plymouth Street**
**Midland Michigan 48640 (US)**
Inventor: **Meyers, Paul A.**
**6536 King Way**
**Dublin California 94568 (US)**

(74) Representative: **Weickmann, Heinrich, Dipl.-Ing. et al**
**Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel Postfach 860820**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 192 068 B1**

## Description

The present invention relates to composite materials comprising synthetic biodegradable polymers and unsintered calcium phosphate biomaterials and method for use thereof. Said composite materials are useful as hard tissue prosthetics such as bone prosthetics.

The present invention also relates to an *in situ* polymerization process for the preparation of composite materials comprising an α-amino acid polymer and a sintered or unsintered calcium phosphate biomaterial.

Calcium phosphates are known in the art as physiologically acceptable biomaterials potentially useful as hard tissue prosthetics. The most widely studied of these are hydroxyapatite and tricalcium phosphate. When these materials are shaped and made porous they can be used alone or as a supplement or extender with bone for hard tissue prosthetics. Under appropriate conditions and with an appropriate form of calcium phosphate, the calcium phosphate is resorbed and new bone growth results. Calcium phosphate biomaterials can be molded by compaction under high pressure. Pore formation of molded calcium phosphate biomaterials is generally achieved by compaction of calcium phosphate powders containing naphthalene followed by removal of the naphthalene by leaching or sublimation. Hydrothermal exchange of marine coral structures (i.e., calcium carbonate for calcium phosphate), and decomposition of hydrogen peroxide have also been employed to generate pore filled structures.

The dense or "green" forms of the calcium phosphate implant materials have mechanical properties equal to or exceeding that of natural bone, but their respective porous forms do not, thus severely limiting their usefulness as hard tissue prosthetics.

The art teaches that natural and synthetic polymers can be used in conjunction with various inorganic mineral fillers such as porous and powdered forms of calcium phosphate to enhance their mechanical properties for use as hard tissue prosthetics or to enhance the bonding of metallic or plastic prosthetics to natural tissue. Natural polymers include collagen and gelatin. Synthetic polymers include polyacrylates, poly(methylmethacrylate), polyethylene, polysulfones, polyamides, polyesters, polytetrafluoroethylene, and polycarbonates; polyacetates and polyglycolates; epoxides, polyacrylamide, polypropylene, polyurethanes, polyacetils, silicone resins, and furan resins; polyvinyl pyrrolidone, polyvinyl alcohol; and a crosslinked pentapeptide. The natural polymers and some of the synthetic polymers are resorbable, i.e., biodegradable.

The art also teaches that nontoxic water soluble substances such as sodium chloride can be incorporated into a mixture of powdered acrylic polymer, liquid monomer, and other ingredients in a mold and the mixture polymerized to produce a shaped composite. The composite can then be made porous by leaching the sodium chloride with water.

The various polymer-calcium phosphate composites are prepared in a number of ways including blending calcium phosphates with polymeric binder and subsequent molding; impregnation of sintered, porous calcium phosphate with polymers under vacuum; impregnation of a porous calcium phosphate body with the melt or solution of prepolymers and solidifying the polymers by further polymerization or curing in the pores or by evaporation of the solvent; impregnation of a porous calcium phosphate body with a very reactive monomer like an α-cyanoacrylate or monomer and catalyst and polymerizing by heating; compression molding of an intimately blended, finely powdered mixture of polymer and calcium phosphate; and embedding ceramic calcium phosphate particles into resins where the calcium phosphate particles have previously been coated with a resin-affinic material to ensure good bonding to the resin, or copolymerizing precoated particles with the resin monomers.

Calcium phosphate-polymer composite materials can also be used in conjunction with metallic or plastic prosthetics to facilitate adhesion and bone growth around the prosthetic. The composite can also be applied as a coating, for example, to an anodized titamium/aluminium/vanadium alloy hip prosthetic. The essential element in anchoring prosthetic devices appears to be the induction of new bone growth around the device by assuring that contact with the surrounding tissue is through a sheath of, or a surface laden with, bioactive calcium phosphate.

It is desired to have a composite material which is gradually absorbed by the host and is simultaneously replaced with bone tissue without any undesirable side effects such as extensive inflammation or extensive formation of connective tissue. It is also desirable to have a composite material based on calcium phosphates which has improved mechanical properties for use as hard tissue prosthetics over calcium phosphates alone. The prior art teaches composites which are comprised of certain polymers and certain calcium phosphates; however, the calcium phosphates in composites of this type are taught as requiring a sintering step.

We have surprisingly found that the calcium phosphates in composites of synthetic biodegradable polymers and calcium phosphates do not require a sintering step in order for the composites to have the desirable properties described herein. The elimination of the need for sintering the calcium phosphates in the composites of the present invention is a significant improvement over the teachings in the prior art. Sintering involves heating at high temperatures, such as 1000°C to 1300°C, which requires substantial levels of energy. Obviating the need for sintering therefore results in reduced energy consumption. In addition, elimination of a sintering step results in a savings of time. Therefore, the composites of the present invention can be prepared at reduced cost.

The present invention is directed to a composite material of a calcium phosphate biomaterial and an organic material useful as a hard tissue prosthetic characterized in that said composite material is comprised of from 25 percent to 75 percent by weight of unsintered hydroxyapatite, unsintered tricalcium phosphate, an unsintered calcium pyrophosphate and/or mixtures thereof, and from 25 percent to 75 percent by weight of a synthetic biogradable polymer selected from a polyester of lactic acid, a polyester of glycolic acid, polyhydroxybuturate, and an α-amino acid polymer, said percentage by weight being based on the total weight of the calcium phosphate biomaterial plus the organic material, said composite material also containining a water-soluble pore-forming agent, selected from poly(2-ethyl-2-oxazoline), polyvinyl pyrrolidone, polyvinyl alcohol, methylcellulose, sodium chloride or potassium chloride in an amount of 10 to 30 percent by weight, said percent being based on the total weight of the calcium phosphate biomaterial, plus organic material, plus pore-forming agent.

The composite materials of the present invention preferably contain from 40 to 60 percent by weight of the calcium phosphate biomaterial and from 40 to 60 percent by weight of the organic material, said percentages being based on the total weight of the calcium phosphate biomaterial plus the organic material.

It is also preferred that said composite materials contain from 10 to 20 percent by weight of the pore-forming agent, said percent being based on the total weight of the calcium phosphate biomaterial plus organic material plus pore-forming agent.

The present invention is also directed to a process for preparing composite material containing from 25 percent to 75 percent by weight of sintered or unsintered calcium phosphate biomaterial and 25 percent to 75 percent by weight of an α-amino acid polymer, said percentages being based on the total weight of the calcium phosphate biomaterial plus α-amino acid polymer, characterized in that said process comprises polymerizing *in situ* an N-carboxyanhydride of an α-amino acid in the presence of a powdered calcium phosphate biomaterial, said polymerization taking place in an inert organic solvent selected from chloroform, dioxane, tetrahydrofuran, methylene chloride, and mixtures thereof, and adding a pore-forming agent, selected from poly(2-ethyl-2-oxazoline), polyvinyl pyrrolidone, polyvinyl alcohol, methylcellulose, sodium chloride or potassium chloride, to the composite material prepared by said *in situ* polymerization.

Suitable synthetic biodegradable polymers for use in the composites of this invention can be made by the polymerization of monomers such as lactic acid, glycolic acid, hydroxybutyrate, amino acids, and the like. Experiments of preferred polymers are polyesters of lactic acid, polyesters of glycolic acid, and polyhydroxybutyrate. Most preferred are polymers of α-amino acids. For the purpose of this invention, the term "synthetic biodegradable polymer" includes copolymers which are the polymerization product of at least two of the hereinabove described monomers and further includes mixtures of the hereinabove described polymers.

The calcium phosphates of the composites of this invention may be one or more unsintered calcium phosphates such as, for example, calcium phosphate tribasic ($Ca_{10}(OH)_2(PO_4)_6$) also known as hydroxyapatite or simply apatite; unsintered tricalcium phosphate ($Ca_3(PO_4)_2$); various unsintered calcium pyrophosphates or mixtures thereof. Preferable calcium phosphate biomaterials are unsintered hydroxyapatite, unsintered tricalcium phosphate or mixtures thereof.

In preparing the composites of this invention, the synthetic biodegradable polymer can be prepolymerized and mixed with powdered unsintered calcium phosphate or the composite can be prepared by impregnating pre-formed unsintered calcium phosphate with monomer, prepolymer or polymer followed by polymerization if necessary. The most preferred method of preparing the composites of this invention is *in situ* polymerization of monomer in the presence of the powdered unsintered calcium phosphate. The *in situ* polymerization is another aspect of the present invention which will be described in detail hereinafter. The *in situ* process is not limited to unsintered calcium phosphate, that is, said process is also applicable to sintered calcium phosphates as well. The composite prepared by the *in situ* polymerization process as described hereinafter that do not contain sintered calcium phosphate are within the scope of the composite materials of the present invention.

The composite materials of the present invention may be ground to find, free-flowing powders making them convenient to use. The free-flowing powders can be readily molded to virtually any shape, preferably a shape capable of anatomical use as a prosthetic device.

Such anatomically-shaped forms may then be surgically implanted into animals in need of such prostheses thereby providing supplementation of replacement of hard tissue. It is further contemplated that the composite materials described herein may be used in conjunction with conventional prosthetic devices known to the art. For instance, in total hip joint replacements, it would be possible to mold one or more of the composite materials described herein about the metal stem of the prosthetic which, when implanted into the femur, would present a compatible surface for new bone growth while being sufficiently strong to support the metal prosthesis. This and other applications of the technology disclosed herein will be readily appreciated by one skilled in the art.

Porositization of the composite materials described herein is attained by intimately blending the powdered composite with a compatible, pore-forming agent selected from poly(2-ethyl-2-oxazoline), hereinafter referred to as PEOX; polyvinyl pyrrolidone; polyvinyl alcohol; or methylcellulose which are water-soluble polymers and/or the water-soluble inert materials sodium chloride or potassium chloride.

The mixture obtained may then be molded to the desired configuration, followed by a leaching of the compatible, pore-forming agent with water. Said leaching typically occurs satisfactorily in a time from 2 to 21 days. Preferably, for the porositization process, PEOX or sodium chloride is utilized as the pore-forming agent. Sodium chloride is particularly preferred as a pore-forming agent.

The composite material disclosed herein may be used in a variety of applications such as, for example, hard tissue prosthetics for dental or orthopedic appliances or other applications where one skilled in the art would envision the use of physiologically acceptable and/or resorable materials such as those described herein.

The composite material described herein is subsequently made porous in order to facilitate tissue ingrowth, a phenomenon where tissue such as bone and tendon continue to grow after the prosthetic device is in place and occupy apertures adjacent to the tissue. The tissue ingrowth provides a means by which a prosthetic device may be secured, thereby providing mechanical stabilization of the implant.

The preferred composites containing α-amino acid polymers prepared as described herein are permeable to oxygen and water and are biodegradable, presumably due to the presence of peptide bonds in the α-amino acid polymer matrix making the substances protein-like. As resorption of the calcium phosphate biomaterial occurs followed by a slow degradation of the α-amino acid polymer matrix, further porositization results, thereby facilitating tissue ingrowth. For example, as the calcium phosphate biomaterial is resorbed and the α-amino acid polymer matrix slowly degrades, new, natural, self-supporting hard tissue develops.

Further, various combinations of α-amino acids may be polymerized with one or more calcium phosphate biomaterials. By so doing, the characteristics of the resulting composite material may be modified so as to vary the rate of resorption of the inorganic filler and/or the rate of degradation of the polymer matrix, thus allowing one skilled in the art to design a given composite for a highly specific application.

The α-amino acids used in the process of the present invention may be any of the common, naturally occurring or synthetic α-amino acids capable of undergoing polymerization through the corresponding reactive α-amino acid N-carboxyanhydride monomer (for convenience, hereinafter referred to as α-amino acid NCA).

Examples of the α-amino acids which may be used include compounds such as aspartic acid, glutamic acid, lysine, arginine, alanine, valine, leucine, serine and the like. The α-amino acids used herein may be present in the D or L configuration or in the D,L configuration.

It is necesary to insure that during the α-amino acid NCA polymerization no side chain reactions or interactions between amino and carboxyl functions of different amino acids occur. Such situations may be prevented by carrying out the reaction in such a way as to avoid said interactions or by using α-amino acids wherein protecting groups have been added to the side chain, amino and/or carboxyl functions. Amino acids having such protected functionalities are readily prepared by known techniques or are commercially available.

Of the α-amino acids which may be used in the present invention, glutamic acid is preferred. Glutamic acid may be polymerized by known techniques without the addition of the above-described protecting groups, or derivatives of glutamic acid may be used. Especially preferred for use herein are the γ-ester derivatives of glutamic acid of the formula:

$$ROOC{-}CH_2{-}CH_2{-}\underset{\displaystyle NH_2}{\underset{|}{CH}}{-}COOH \qquad (I)$$

wherein R represents alkyl or aralkyl. As used herein, the term "alkyl" refers to aliphatic, straight or branched chain radicals of from 1 to 10 carbon atoms or cyclic aliphatic radicals of from 3 to 8 carbon atoms; "aralkyl" refers to radicals such as phenylethyl, benzyl, and ring-substituted benzyl. Most particularly preferred for use herein are those compounds of formula I wherein R is methyl or benzyl.

The α-amino acid NCA referred to above is prepared by the reaction of the desired α-amino acid with

phosgene via procedures known to the art. For purposes of illustration, the N-carboxyanhydride of a compound of formula I is prepared by the following reaction sequence (where R is as defined for formula I):

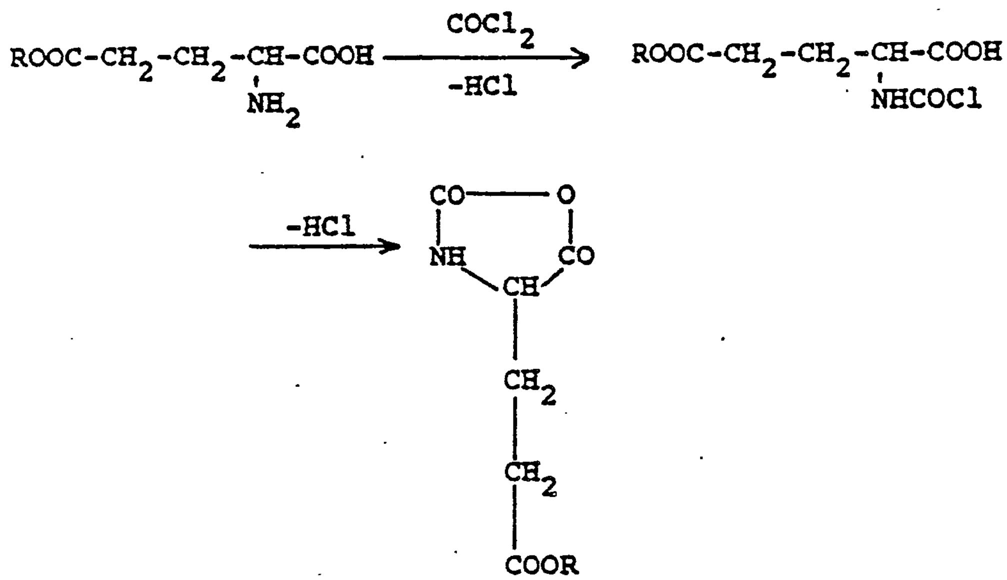


The α-amino acid NCA is then readily polymerized into the α-amino acid polymer as represented by the following:

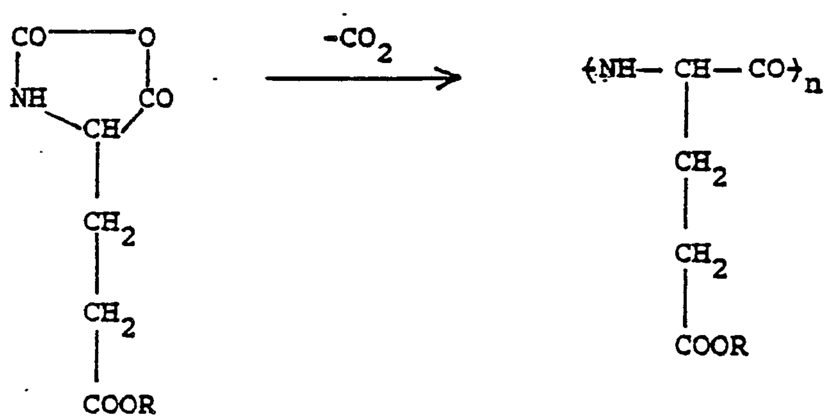


wherein R is as defined for Formula I and n is a positive integer. The other α-amino acid polymers alluded to herein may be prepared in a manner analogous to the above-described reactions; the use of compounds of Formula I is merely illustrative. Further, one skilled in the art will appreciate that α-amino acid polymers may be prepared by techniques other than as described herein (i.e., by processes other than polymerization of an α-amino acid NCA monomer) such as by the use of active esters of triphenylphosphite with imidazole and the like.

In the presence of the present invention, the α-amino acid NCA is polymerized in the presence of one or more sintered or unsintered calcium phosphate biomaterials. The calcium phosphate biomaterial may be, for example, sintered or unsintered calcium phosphate tribasic ($Ca_{10}(OH)_2(PO_4)_6$ also known as hydroxyapatite or simply apatite, tricalcium phosphate ($Ca_3(PO_4)_2$), various calcium pyrophosphates or mixtures thereof. The composite materials thus formed contain the same properties of calcium phosphate and α-amino acid polymer as described hereinabove.

In preparing the reactive α-amino acid NCA monomer used in the *in situ* process of the present invention, the desired α-amino acid (having, if necessary, protected side chain, amino and/or carboxyl functionalities) is treated with phosgene. While various phosgenation processes are known to the art, it is preferable that a process substantially the same as that described in U.S. Patent No. 3,658,831 and illustrated in the present examples be utilized in order to prepared an α-amino acid NCA of the desired purity. It is important to obtain very highly pure α-amino acid NCA in order to prepare α-amino acid polymers having a high degree of polymerization and high quality.

The α-amino acid NCA thus obtained is then admixed with one or more of the desired calcium phosphate biomaterials in a suitable inert organic solvent such as chloroform, dioxane, tetrahydrofuran

(THF), methylene chloride or mixtures thereof. Preferably, the inert organic solvent utilized is dioxane, THF or mixtures thereof. Preferably, the inert organic solvent utilized is dioxane, THF or mixtures thereof. The calcium phosphate biomaterial must be in a powdered or particulate form. Typically the calcium phosphate particles are between 0.05 micrometers (μm) and 10 μm in diameter and preferably about 1 μm in diameter. As noted earlier, the composite material may be composed of from 25 to 75 percent by weight, preferably from 40 to 60 percent by weight of one or more calcium phosphate biomaterials, preferably hydroxyapatite, tricalcium phosphate, or mixtures thereof, said percentages being based on total calcium phosphate plus α-amino acid polymer. Correspondingly, the α-amino acid polymer represents from 75 to 25 percent by weight, preferably from 60 to 40 percent by weight of the composite formed, said percentages being based on the total calcium phosphate plus α-amino acid polymer. Typically, the α-amino acid NCA and calcium phosphate biomaterial mixture is stirred for a period of time sufficient to effect formation of the desired composite material (usually from 2 to 12 days) at a temperature of from 18° to 30°C. It is preferred that the mixture be stirred for 3 to 6 days at ambient temperature and pressure.

The above-described *in situ* process of the present invention is a significant improvement of the processes taught in the prior art. The *in situ* process of the present invention does not require exogenous catalysts or initiators for polymerization of the α-amino acid NCA. The polymerization of the α-amino acid NCA is catalyzed by the surfaces of the calcium phosphate particles. Further, the surfaces of the calcium phosphate particles do not require the presence of resins or other coupling agents. The calcium phosphate particles do not have to be surface modified prior to the *in situ* polymerization of the α-amino acid NCA. In addition, the *in situ* polymerization process of the present invention proceeds spontaneously at ambient temperature without the need for heating or cooling. Also, it is unnecessary to use a solvent system in which both the α-amino acid NCA monomer and resultant α-amino acid polymer are soluble. For example, poly(γ-methyl)-L-glutamate is insoluble in dioxane or THF, two solvents frequently used for the polymerization. The *in situ* process of the present invention is less complicated, is less costly, and requires fewer steps than the prior art processes.

In addition to the hereinabove described advantages, the *in situ* process of the present invention results in an intimate bonding between the resulting α-amino acid polymer and calcium phosphate biomaterial, not merely a mixture of said components. This intimate bonding is not achieved in more complicated processes employing exogenous catalysts or processes which require surface modification of the calcium phosphate particles. The *in situ* polymerization process reults in maximum contact between the polymer and calcium phosphate. The polymer is in a continuous phase which coats the calcium phosphate particles; this results in relatively constant and uniform dispersion of the calcium phosphate particles in the polymer matrix. Once the preferred composite material has been prepared it can be molded by techniques well-known in the art to virtually any desired shape while maintaining the complete integrity of the composite material. The composites are porositized by using the pore-forming agents at the concentration described herein, using the techniques described herein.

The following examples are provided as a means of illustrating the present invention and are not to be construed as a limitation thereon.

## Example 1

γ-Benzyl L-Glutamate

1416 grams (g) of L-glutamic acid, 1560 g of 60% sulfuric acid and 1152 g of benzyl alcohol were placed in a 12 liter round bottom flask equipped with a distillation head. The mixture washeated to 70°C and stirred for 45 minutes. Upon cooling, the resulting solution was stirred and was subjected to a reduced pressure. When the vacuum had stabilized at about 100 millimeters (mm) the reaction temperature was again raised to 70°C and water was distilled off for about 4.5 hours. Upon standing overnight, the reaction mixture became viscous and was slowly added to a stirred mixture of 1613 g of sodium bicarbonate, 1.2 kilograms (kg) of ice and 12 liters of water. A precipitate formed which was collected by filtration and subsequently washed with about 8 liters of carbon dioxide-free water and 8 liters of acetone and subsequently air-dried. The precipitate was triturated with 2 liters of ether and dried, yielding 1044 g of the desired γ-benzyl L-glutamate, melting point (m.p.) 156°—157°C. Thin layer chromatography detected the presence of unreacted glutamic acid in the crude product. The crude product was recrystallized from 12.5 liters of hot water and filtered through a plug of glass wool suspended in the neck of a heated glass funnel. After cooling, and overnight refrigeration, the recrystallized product was collected, and washed with 2 liters of cold water, then 2 liters of THF. The product was air dried overnight and then dried *in vacuo* at room temperature for three hours. 693 g of γ-benzyl L-glutamate was recovered as white, shiny plates, m.p. 156.5°—157°C.

Following a procedure substantially the same as that described in Example 1, the following two compounds were prepared using the requisite starting materials.

## Example 2

γ-Benzyl D,L-Glutamate, m.p. 145°—146°C.

## Example 3

γ-Hexyl L-Glutamate, m.p. 162.5°—163°C.

Example 4

γ-Methyl L-Glutamate

A cold solution of 300 ml of acetyl chloride was slowly added to a flask containing 3 liters of methanol. To this mixture was added 442 g of L-glutamic acid. The flask was stoppered and shaken for several minutes to effect solution. The flask was then allowed to stand at room temperature with intermittent shaking for 24 hours. 300 ml of pyridine was added causing a precipitate to form. Upon standing for an additional 48 hours, the precipitate was collected on sintered glass and washed with two 600 ml portions of ethanol and a 250 ml portion of ether. The precipitate was dried *in vacuo* at room temperature for 3 hours and then in a vacuum desiccator over anhydrous calcium sulfate (Drierite®) for 5 hours. Pyridine vapors were still perceptible from the precipitate which was further triturated with ether and dried again yielding 201.5 g of the desired γ-methyl L-glutamate as white, shiny plates, m.p. 168°—169°C.

Example 5

γ-Methyl D,L-Glutamate

γ-Methyl D,L-glutamate was prepared by substantially the same method as described in Example 4, yielding white, powder-like crystals, m.p. 166°—166.3°C.

Example 6

γ-Benzyl L-Glutamate N-Carboxyanhydride

92.7 g of γ-benzyl L-glutamate and 840 ml of THF were mixed and heated in a 3 liter reaction flask. Nitrogen and phosgene were bubbled in and the reaction temperature was maintained between 45°—50°C until complete solution of the starting material had occurred (about 2 hours). Heating and phosgene flow were then stopped, but stirring and nitrogen flow were continued as the reaction mixture cooled slowly to 30°C (approximately 45 minutes). The reaction flask was carefully removed from the phosgenation apparatus and stoppered. The reaction mixture was then concentrated *in vacuo* to about 250 ml with the aid of a rotary evaporator (maximum bath temperature about 35°C). The residual concentrate was transferred to a dry flask and diluted carefully with an equal volume of hexane and seeded. After allowing crystallization to proceed at room temperature for about an hour, the reaction mixture was further diluted with about 500 ml of hexane and was maintained at −30°C for about 8—10 hours. After warming to a room temperature the product was collected on a sintered glass funnel, care being taken to minimize contact with atmospheric moisture. The product was rinsed with a mixture of THF-hexane (1:3) and then hexane, covered with a filter paper and dried in a vacuum desiccator over anhydrous calcium sulfate (Drierite®). 92.6 g of the desired γ-benzyl L-glutamate N-carboxyanhydride was recovered as white crystals, m.p. 95°—96°C.

Example 7

γ-Methyl L-Glutamate N-Carboxyanhydride

100 g of γ-methyl L-glutamate and 600 ml of THF were placed in a 2 liter flask under nitrogen. The ensuing phosgenation reaction was carried out as described in Example 6, above. The reaction temperature was maintained between 44°—49°C for about 3 hours. Heating and phosgene addition were discontinued and stirring of the reaction mixture under nitrogen continued for about 1 hour before working up. 93.9 g of the desired γ-methyl L-glutamate N-carboxyanhydride was recovered as dense, white crystals, m.p. 97.5°—99°C.

Example 8

Tricalcium Phosphate

Tricalcium phosphate (β-whitlockite crystalline form) was prepared by the following technique. A solution of 141.7 grams of calcium nitrate tetrahydrate in 400 ml of water was prepared and the pH adjusted to about pH 11 with concentrated ammonium hydroxide. This solution was then diluted to about 900 ml with water and placed in a three liter flask fitted with a dropping funnel and mechanical stirring apparatus. Separately, 66.1 grams of ammonium phosphate dibasic was added to 750 ml of water. The pH of the resulting solution was adjusted to about pH 11 with concentrated ammonium hydroxide resulting in the formation of a precipitate which was subsequently dissolved by the addition of water (about 2000 ml total volume of the solution). The ammonium phosphate dibasic solution was then slowly added to the reaction flask containing the calcium nitrate tetrahydrate solution and the resulting mixture was stirred overnight. A precipitate formed which was collected by centrifugation, washed with water and again collected by centrifugation. The precipitate was then suspended in a 2 percent aqueous ammonium sulfate solution and filtered leaving a residue which was subsequently dried *in vacuo* at 90°C leaving 63.7 g of tricalcium phosphate. The tricalcium phosphate was then sintered at 1150°C for one hour, and then ground to a fine powder.

Example 9

Hydroxyapatite-Poly(γ-Methyl-L-Glutamate) Composite

5.0 g of γ-methyl L-glutamate N-carboxyanhydride was added to 50 ml of a mixture of dioxane-THF (3:1). Upon solubilization, 5.9 g of dry unsintered calcium phosphate tribasic (i.e., hydroxyapatite) was

added and the mixture was stirred at room temperature for seven days. The mixture was then poured with stirring into 300 ml of methanol and the product composite was collected by filtration, washed with methanol and dried *in vacuo* at 80°C for 6 hours. 9.58 g of a soft, white, homogeneous solid was obtained and subsequently identified as hydroxyapatite-poly(γ-methyl L-glutamate) composite consisting of 61% (by weight) hydroxyapatite. This composite material was easily ground to a fine powder.

Example 10

Hydroxyapatite-Poly(γ-Methyl L-Glutamate) Composite

Following a procedure substantially the same as that described in Example 9, 65.2 g of γ-methyl L-glutamate N-carboxyanhydride, 50 g of unsintered hydroxyapatite and 675 ml of a mixture of dioxane-THF (3:1) were stirred continuously for 5 days. Two liters of methanol were then added to the mixture and the desired composite material was recovered as described in Example 9. 98 g of the desired hydroxyapatite-poly(γ-methyl L-glutamate) composite material consisting of 50% (by weight) hydroxyapatite was subsequently recovered.

Example 11

Hydroxyapatite-Poly(γ-Benzyl L-Glutamate) Composite

72.6 g of γ-benzyl L-glutamate N-carboxyanhydride, 40 g of unsintered hydroxyapatite, and 700 ml of a mixture of dioxane-THF (3:1) were continuously stirred for four days. The reaction mixture was then poured with stirring into 2500 ml of ethanol and collected by filtration. The residue from the filtration was washed with ethanol, air dried and then dried *in vacuo* at 60°—70°C for six hours. 98 g of the desired hydroxyapatite-poly(γ-benzyl L-glutamate) composite material (60 percent by weight hydroxyapatite) was obtained as a white, short fiber-like solid.

Example 12

Tricalcium Phosphate-Poly(γ-Benzyl L-Glutamate) Composite

γ-Benzyl L-glutamate N-carboxyanhydride (2.05 g) and sintered tricalcium phosphate (1.14 g) were combined in 40 ml of a mixture of dioxane-THF (3:1) and continuously stirred for 9 days. The resulting composite material was collected by pouring the reaction mixture into 200 ml of ethanol (with stirring) followed by filtration on a fritted glass filter leaving a solid residue. The residue was washed with ethanol and dried *in vacuo* to give 2.73 g of the desired tricalcium phosphate-poly(γ-benzyl L-glutamate) composite material (60 percent by weight tricalcium phosphate).

Example 13

Tricalcium Phosphate-Poly(γ-Methyl L-Glutamate) Composite

γ-Methyl L-glutamate N-carboxyanhydride (129.3 g) was dissolved in 1600 ml of a mixture of dioxane-THF (3:1). While maintaining a positive nitrogen flow through the system, 98.9 g of sintered tricalcium phosphate was added and the resulting mixture was continuously stirred for 12 days. The composite material thus formed was collected by pouring the reaction mixture into about 1500 ml of methanol followed by filtration on a fritted glass filter leaving a solid residue. The residue was washed with three 500 ml portions of methanol and then dried *in vacuo* at 70°C for 20 hours to give 194.2 g of the desired tricalcium phosphate-poly(γ-methyl L-glutamate) composite material (50 percent by weight tricalcium phosphate) as a soft, white, powdery material.

Example 14

A Hydroxyapatite-poly(γ-benzyl L-glutamate) composite was ground and sieved through a 20-mesh screen. PEOX (molecular weight about 200,000) was ground and sieved through a 35-mesh screen. Enough PEOX was added to the ground composite to constitute 15 percent by weight of the total mixture (i.e., composite plus PEOX) and the mixture was blended by tumbling on a roller for 2 hours. This mixture was then compression molded in nickel plated stainless steel pressurized dies held in a ram press under a 2267.96 kilogram (2.5 ton) load at 160°C. One die was cylindrical in shape and produced a pressure of 175816.3 kilopascals (kPa) (25,500 pounds per square inch (psi)) at the composite surface. Another die was dogbone shaped, producing a pressure of 17926.37 kPa (2600 psi) at the composite surface. Molding time was 20 minutes, with about 10 minutes additional being allowed for the preheated press and die to approach the desired molding temperature. About 3—3.5 g of the composite-PEOX mixture was used to produce a molded, 1.27 centimeter (0.5 inch) disc in the cylindrical die. Similarly, about 7—7.5 g of the composite-PEOX mixture was used to produce 0.3175 centimeter (0.125 inch) thick bars in the dogbone shaped die. The molded products were white, smooth, homogeneous objects.

Example 15

The same procedure used in Example 14 was repeated utilizing a hydroxyapatite-poly(γ-methyl L-glutamate) composite and PEOX mixture. The mixture was molded in the dies described above under 2267.96 kilogram (2.5 ton) load at 220°C. The molded products were white, smooth, homogeneous objects.

Example 16

Tricalcium phosphate-poly(γ-methyl L-glutamate) composite material was ground and sieved through

a 35 mesh screen. Enough sodium chloride was added to the ground composite to constitute 15 percent by weight of the total mixture (i.e., composite plus sodium chloride). The mixture was blended by tumbling on a roller overnight and was subsequently molded as described in Example 15.

Similarly, tricalcium phosphate-poly(γ-benzyl L-glutamate) composite material was admixed with a sufficient quantity of sodium chloride to constitute 15 percent sodium chloride by weight of the total mixture (i.e., composite plus sodium chloride). The mixture was blended and then molded as described in Example 14.

Example 17

The same procedures utilized in Examples 14 and 15 were again repeated using sodium chloride in place of PEOX as the pore-forming agent. Again, the resulting molded products were white, smooth, homogeneous objects.

Example 18

In order to illustrate the porositization technique, a 2.934 g molded disc containing 87 percent hydroxyapatite-poly(γ-methyl L-glutamate) composite material (50 percent by weight of each constituent) and 13 percent PEOX blended therein was placed in 20 ml of water in a closed container for a total of six days (the water was changed after four days). The disc was removed and dried by blotting with absorbent paper and then further dried in an oven at 60°C. 0.325 g of weight was lost representing 85.3 percent of the available PEOX in the molded disc. Microscopy of a section of the porositized product showed pore sizes of 10—25 microns.

Utilizing the above procedure, a molded disc containing 85 percent hydroxyapatite-poly(γ-methyl L-glutamate) composite (50 percent by weight of each component) and 15 percent by weight sodium chloride blended therein was placed in water in a closed container (48 percent of the available sodium chloride was removed). Microscopy of a section of the disc showed varied pore sizes, some in excess of 100 microns.

Similarly, a molded bar weighing 9.92 g containing 85 percent by weight tricalcium phosphate-poly(γ-methyl L-glutamate) composite (50 percent by weight of each constituent) blended with 15 percent by weight sodium chloride was placed in 250 ml of water in a closed container. After 10 days, the bar was removed, dried *in vacuo* for 7 hours at 90°C and weighed. 77.3 percent (1.15 g) of the available sodium chloride had been removed.

The process of the present invention can be used to prepare composites such as those described herein having desirable properties. Utilizing procedures described herein, additional composite materials set forth in Table 1 were prepared. Table 2 describes the mechanical properties of various molded composite materials.

**TABLE 1**

| Example No. | Composite Material: Polymer[a] | Composite Material: Calcium Phosphate Biomaterial[b] (Weight %) | Porositization Mode: Agent (Amount)[c] | Porositization Mode: Amount Removed[d] |
|---|---|---|---|---|
| 19 | PGMLG | HA (50) | None | - |
| 20 | PGMLG | HA (50) | None | - |
| 21 | PGMLG | HA (50) | PEOX (15) | 80 |
| 22 | PGMLG | HA (50) | PEOX (15) | 122[e] |
| 23 | PGMLG | HA (50) | NaCl (15) | 48 |
| 24 | PGBLG | HA (40) | None | - |
| 25 | PGBLG | HA (40) | None | - |
| 26 | PGBLG | HA (40) | PEOX (15) | 29 |
| 27 | PGBLG | HA (40) | PEOX (15) | 59 |
| 28 | PGMLG | TCP (50) | NaCl (15) | 63 |
| 29 | PGMLG | TCP (50) | None | - |
| 30 | PGMLG | TCP (50) | NaCl (15) | 65 |
| 31 | PGMLG | TCP (50) | None | - |
| 32 | PGMLG | HA (50) | NaCl (10) | 55 |
| 33 | PGMLG | HA (50) | NaCl (20) | 40 |
| 34 | PGMLG | HA (75) | NaCl (15) | 78 |

[a]Abbreviations: PGMLG = poly(γ-methyl L-glutamate)
PGBLG = poly(γ-benzyl L-glutamate)

[b]Abbreviations: HA = hydroxyapatite
TCP = tricalcium phosphate

[c]Weight percent of the porositizing agent based on the total weight of the composite material plus porositizing agent.

[d]Refers to the percent by weight of porositizing agent removed (based on the theoretical amount of porositizing agent available) by leaching with water.

[e]Result due to error in weighing.

**TABLE 2**

**MECHANICAL PROPERTIES OF VARIOUS COMPOSITE MATERIALS**

| Composite of Example No. | Vicat Heat Distortion | Compression Data: Strength (psi) kPa | Compression Data: Deformation (%) | Compression Data: Recovery[a] (%) | Compression Data: Modulus (psi x $10^5$) kPa x $10^6$ |
|---|---|---|---|---|---|
| 19 | - | (12,380) 85357.1 | 0.20 | - | (4.28) 2.95 |
| 20 | 230°C | - | - | - | - |
| 21 | - | (5,177) 35694.2 | - | - | - |
| 22 | 230°C | - | - | - | - |
| 23 | - | (9,029) 62252.8 | 3.6 | 27.8 | (3.75) 2.59 |
| 24 | - | (3,809) 26262.1 | 1.25 | 42.4 | (1.78) 1.23 |
| 25 | 99°C | - | - | - | - |
| 26 | - | (3,542) 24421.2 | - | - | - |
| 27 | 91°C | - | - | - | - |
| 28 | - | (4,824) 33260.3 | - | - | (3.13) 2.16 |
| 29 | - | (8,034) 55392.5 | 3.61 | 31.8 | (3.77) 2.60 |
| 34 | - | (8,017) 55275.3 | 1.26 | 20.0 | (4.30) 2.96 |

a = After 24 hours.

Example 35

Molded discs containing 85 percent hydroxyapatite-poly(γ-methyl L-glutamate) composite (50 percent by weight of each component) and 15 percent sodium chloride blended therein (hereafter referred to as the 15 percent NaCl composite discs) were porositized by the general techniques described herein. Similarly, molded discs containing 80 percent hydroxyapatite-poly(γ-methyl L-glutamate) composite (50 percent by weight of each component) and 20 percent sodium chloride blended therein (hereafter referred to as the 20 percent NaCl composite discs) were also prepared and porositized for evaluation in the study described below.

Small pieces of the above-described molded composites were cut from the larger discs with a diamond blade and subsequently autoclaved for sterilization. The sterilized pieces were then surgically implanted into rabbits in an incision in the paravertebral muscles of the lumbar region and in a hole drilled in the iliac crest. X-rays taken 6 and 12 weeks later of the rabbits implanted with the 15 percent NaCl composite discs showed no remarkable soft-tissue response in the lumbar paravertebral muscles indicating absence of a chronic inflammatory reaction. The bony implant sites exhibited healing and a regeneration of new bone incorporating the surgically implanted composite material. The animals were sacrificed at 14 weeks and the discs and surrounding musculature of the paravertebral implant were excised for microscopic evaluation. Likewise, iliac crest sections containing the implant sites were surgically removed, decalcified and were subsequently made into paraffin embedded sections. Upon examination, the discs showed a thin, fibrous encapsulation of from 10 to 50 microns evidencing only a minor foreign body response.

Animals implanted with the 20 percent NaCl composite discs showed a similar clinical history except that eight weeks after implantation there was no fibrous capsule formation around the implants and marrow was observed growing into the pores of the discs.

**Claims**

1. A composite material of calcium phosphate biomaterial and an organic material useful as a hard tissue prosthetic characterized in that said composite material is comprised of from 25 percent to 75 percent by weight of unsintered hydroxyapatite, unsintered tricalcium phosphate, an unsintered calcium pyrophosphate and/or mixtures thereof, and from 25 percent to 75 percent by weight of a synthetic biodegradable polymer selected from a polyester of lactic acid, a polyester of glycolic acid, polyhydroxybutyrate, and an α-amino acid polymer, said percentage by weight being based on the total weight of the calcium phosphate biomaterial plus the organic material, said composite material also containing a water-soluble pore-forming agent, selected from poly(2-ethyl-2-oxazoline), polyvinyl pyrrolidone, polyvinyl alcohol, methylcellulose, sodium chloride or potassium chloride in an amount of 10 to 30 percent by weight, said percent being based on the total weight of the calcium phosphate biomaterial, plus organic material, plus pore-forming agent.

2. The composite material of Claim 1 containing from 40 to 60 percent by weight of the calcium phosphate biomaterial, from 40 to 60 percent by weight of the organic material, said percentages being based on the total weight of the calcium phosphate biomaterial plus the organic material, said composite material also containing from 10 to 20 percent by weight of the pore-forming agent said percent being based on the total weight of the calcium phosphate biomaterial, plus organic material, plus pore-forming agent.

3. The composite material of Claim 1 or 2 wherein the calcium phosphate biomaterial is unsintered hydroxyapatite, the synthetic biodegradable polymer is a glutamic acid-γ-ester, and the pore-forming agent is poly(2-ethyl-2-oxazoline) or sodium chloride.

4. A process for preparing a composite material containing from 25 percent to 75 percent by weight of sintered or unsintered calcium phosphate biomaterial and 25 percent to 75 percent by weight of an α-amino acid polymer, said percentages being based on the total weight of the calcium phosphate biomaterial plus α-amino acid polymer, characterized in that said process comprises polymerizing *in situ* an N-carboxyanhydride of an α-amino acid in the presence of a powdered calcium phosphate biomaterial, said polymerization taking place in an inert organic solvent selected from chloroform, dioxane, tetrahydrofuran, methylene chloride, and mixtures thereof, and adding a pore-forming agent, selected from poly(2-ethyl-2-oxazoline), polyvinyl pyrollidone, polyvinyl alcohol, methylcellulose, sodium chloride or potassium chloride, to the composite material prepared by said *in situ* polymerization.

5. The process of Claim 4 wherein said N-carboxyanhydride is glutamic acid N-carboxyanhydride and said calcium phosphate biomaterial is sintered or unsintered hydroxyapatite, tricalcium phosphate, or mixtures thereof.

6. The process of Claim 4 or 5 wherein said composite material contains from 40 to 60 percent by weight of a calcium phosphate biomaterial and from 40 to 60 percent by weight of an α-amino acid polymer.

**Patentansprüche**

1. Verbundwerkstoff aus Calciumphosphat-Biomaterial und einem organischen Material zur Verwendung als Hartgewebeprothese, dadurch gekennzeichet, daß sich der Verbundwerkstoff zusammensetzt aus 25 bis 75 Gew.% ungesintertem Hydroxyapatit, ungesintertem Tricalciumphosphat, einem ungesinterten Calciumpyrophosphat und/oder Mischungen davon, und aus 25 bis 75 Gew.% eines synthetischen bioabbaubaren Polymers ausgewählt aus einem Milchsäure-Polyester, einem Glycolsäure-Polyester, Polyhydroxybutyrat, und einem α-Aminosäure-Polymer, wobei sich die Gew.% auf das Gesamtgewicht des Calciumphosphat-Biomaterials und des organischen Materials beziehen, und wobei der Verbundwerkstoff auch ein wasserlösliches, porenbildendes Mittel enthält, ausgewählt, aus Poly(2-ethyl-2-oxazolin), Polyvinylpyrrolidon, Polyvinylalkohol, Methylcellulose, Natriumchlorid oder Kaliumchlorid in einer Menge von 10 bis 30 Gew.%, wobei sich die Prozentangabe auf das Gesamtgewicht aus dem Calciumphosphat-Biomaterial, dem organischen Material und dem porenbildenden Mittel bezieht.

2. Verbundwerkstoff nach Anspruch 1, dadurch gekennzeichnet, daß er 40 bis 60 Gew.% Calciumphosphat-Biomaterial und 40 bis 60 Gew.% organisches Material enthält, wobei sich die Prozentangaben auf das Gesamtgewicht aus Calciumphosphat-Biomaterial und dem organischen Material beziehen, und wobei der Verbundwerkstoff auch 10 bis 20 Gew.% des porenbildenden Mittels enthält, wobei sich die Prozentangabe auf das Gesamtgewicht aus dem Calciumphosphat-Biomaterial, dem organischen Material und dem porenbildenden Mittel bezieht.

3. Verbundwerkstoff nach Anspruch 1 oder 2, dadurch gekennzeichnet, daß das Calciumphosphat-Biomaterial ungesinterter Hydroxyapatatit ist, das synthetische bioabbaubare Polymere ein Glutaminsäure-γ-ester ist, und das porenbildende Mittel Poly(2-ethyl-2-oxazolin) oder Natriumchlorid.

4. Verfahren zur Herstellung eines Verbundwerkstoffes, der 25 bis 75 Gew.% gesintertes oder ungesintertes Calciumphosphat-Biomaterial und 25 bis 75 Gew.% eines α-Aminosäure-Polymers enthält, wobei die Prozentangaben sich auf das Gesamtgewicht des Calciumphosphat-Biomaterials und des α-Aminosäure-Polymers beziehen, dadurch gekennzeichnet, daß dieses Verfahren eine *in situ* Polymerisation eines N-Carboxyanhydrids einer α-Aminosäure in Gegenwart eines gepulverten Calciumphosphat-Biomaterials umfaßt, wobei die Polymerisation in einem inerten organischen Lösungsmittel stattfindet, das ausgewählt ist aus Chloroform, Dioxan, Tetrahydrofuran, Methylenchlorid, and Mischungen davon, und die Zugabe eines porenbildenden Mittels, ausgewählt aus Poly(2-ethyl-2-oxazolin), Polyvinylpyrrolidon, Polyvinylalkohol, Methylcellulose, Natriumchlorid oder Kaliumchlorid, zu dem durch diese *in situ*-Polymerisation hergestellten Verbundwerkstoff.

5. Verfahren nach Anspruch 4, dadurch gekennzeichnet, daß das N-Carboxyanhydrid Glutaminsäure-N-carboxyanhydrid ist, und das Calciumphosphat-Biomaterial gesinterter oder ungesinterter Hydroxyapatit, Tricalciumphosphat oder Mischungen davon darstellt.

6. Verfahren nach Anspruch 4 oder 5, dadurch gekennzeichnet, daß der Verbundwerkstoff 40 bis 60 Gew.% eines Calciumphosphat-Biomaterials und 40 bis 60 Gew.% eines α-Aminosäure-Polymers enthält.

**Revendications**

1. Matériau composite d'un biomatériau de phosphate de calcium et d'un matériau organique, utile comme prothèse de tissu dur, caractérisé en ce que ledit matériau composite est constitué, pour 25 à 75% en poids, d'hydroxyapatite non frittée, de phosphate tricalcique non fritté, d'un pyrophosphate de calcium non fritté et/ou de leurs mélanges, et, pour 25 à 75% en poids, d'un polymère synthétique biodégradable choisi parmi un polyester d'acide lactique, un polyester d'acide glycolique, un polyhydroxybutyrate, et un polymère d'acide α-aminé, lesdits pourcentages en poids étant rapportés au poids total du biomatériau de phosphate de calcium et du matériau organique, ledit matériau composite contenant également un agent porogène hydrosoluble, choisi parmi le poly(2-éthyl-2-oxazoline), le poly(vinyl-pyrrolidone), le poly(alcool vinylique), la méthylcellulose, le chlorure de sodium et le chlorure de potassium, en une proportion de 10 à 30% en poids, ce pourcentage étant rapporté au poids total du biomatériau de phosphate de calcium, du matériau organique et de l'agent porogène.

2. Matériau composite de la revendication 1, contenant de 40 à 60% en poids de biomatériau de phosphate de calcium, de 40 à 60% en poids de matériau organique, ces pourcentages étant rapportés au poids total du biomatériau de phosphate de calcium et du matériau organique, ledit matériau composite contenant également de 10 à 20% en poids de l'agent porogène, ce pourcentage étant rapporté au poids total du biomatériau de phosphate de calcium, du matériau organique et de l'agent porogène.

3. Matériau composite de la revendication 1 ou 2, dans lequel le biomatériau de phosphate de calcium est de l'hydroxyapatite non frittée, le polymère synthétique biodégradable est un γ-ester d'acide glutamique, et l'agent porogène est du poly(2-éthyl-2-oxazoline) ou du chlorure de sodium.

4. Procédé de préparation d'un matériau composite contenant de 25 à 75% en poids de biomatériau de phosphate de calcium fritté ou mon et de 25 à 75% en poids d'un polymère d'acide α-aminé, ces pourcentages étant rapportés au poids total du biomatériau de phosphate de calcium et du polymère d'acide α-aminé, caractérisé en ce que ledit procédé comporte la polymérisation in situ d'un N-carboxyanhydride d'un acide α-aminé, en présence d'un biomatériau de phosphate de calcium en poudre, ladite polymérisation ayant lieu dans un solvant organique inerte choisi parmi le chloroforme, le dioxane, le tétrahydrofuranne, le chlorure de méthylène et leurs mélanges, et l'addition d'un agent porogène, choisi parmi le poly(2-éthyl-2-oxazoline), le poly(vinyl-pyrrolidone), le poly(alcool vinylique), la méthylcellulose,

le chlorure de sodium et le chlorure de potassium, au matériau composite préparé par ladite polymérisation in situ.

5. Procédé de la revendication 4, dans lequel ledit N-carboxyanhydride est le N-carboxyanhydride d'acide glutamique, et ledit biomatériau de phosphate de calcium est de l'hydroxyapatite, du phosphate tricalcique, ou leurs mélanges, frittés ou non.

6. Procédé de la revendication 4 ou 5, dans lequel ledit matériau composite contient de 40 à 60% en poids d'un biomatériau de phosphate de calcium et de 40 à 60% en poids d'un polymère d'acide α-aminé.